# EUROPEAN PATENT SPECIFICATION

(11) **EP 1 818 046 B1**
(45) Date of publication and mention of the grant of the patent: **02.04.2008**
(21) Application number: 06425078.0
(22) Date of filing: 13.02.2006
(51) Int. Cl.: A61K 9/127

(54) **Drug delivery device**
Vorrichtung zur Freisetzung von Medikamenten
Dispositive pour la liberation des medicaments

(43) Date of publication of application: 15.08.2007
(73) Proprietor: C.R.F. SOCIETÀ CONSORTILE PER AZIONI, 10043 Orbassano (Torino) (IT)
(72) Inventor: Pizzi, Marco, 10153 Torino (IT); Grasso, Valentina, 10041 Carignano Torino (IT); Pullini, Daniele, C.R.F., 10043 Orbassano Torino (IT); Paderi, Marzia, 10127 Torino (IT); Lambertini, Vito Guido, 10049 Giaveno Torino (IT); Li Pira, Nello, 12045 Fossano Cuneo (IT); Innocenti, Gianfranco, 10040 Rivalta Torino (IT); Valerio, Federica, 10043, Orbassano (Torino) (IT)
(74) Representative: Freyria Fava, Cristina

(56) References cited:
- EP-A- 1 593 406
- WO-A-01/56546
- WO-A-03/026618
- DE-A1- 4 201 461
- US-A1- 2003 211 045
- SATO T ET AL: "THE DEVELOPMENT OF ANTICANCER AGENT RELEASING MICROCAPSULE MADE OF FERROMAGNETIC AMORPHOUS FLAKES FOR INTRATISSUE HYPERTHERMIA" IEEE TRANSACTIONS ON MAGNETICS, IEEE SERVICE CENTER, NEW YORK, NY, US, vol. 29, no. 6, 1 November 1993 (1993-11-01), pages 3325-3330, XP000429351 ISSN: 0018-9464

## Description

The present invention concerns an intravascular device that can be injected into the bloodstream of a human or animal body to deliver drugs. In particular, it relates to drug delivery devices including an electric micro- or nano-capacitor.

The purpose of the present invention is to produce a drug delivery device capable of releasing the drug in a controlled fashion.

According to the present invention, this purpose is achieved thanks to the solution described specifically in the attached claims. The claims form an integral part of the technical instruction provided here in regard to the invention.

The invention relates to a device for the delivery of drugs into a human or animal body comprising:
-- a membrane,
-- a micro- or a nano-capacitor and means to generate an electric potential through the micro- or nano-capacitor, and
-- a drug,
where the micro- or nano-capacitor and the drug are contained inside the membrane and the generation of an electric potential/electric field by the micro- or nano-capacitor determines the release, by the membrane, of the drug contained in it.

It is, indeed, known that application of electric fields to a cell membrane generates the phenomenon known as electroporation, that is the opening/rupture of parts of the membrane with consequent release of the material contained inside it. The present invention exploits this phenomenon for the controlled release of at least one drug contained inside the device.

EP 1593406 discloses nano capacitors of the piezo electric typ serving as carriers for active agents without a membrane.

The invention will now be described in detail, as a simple example without limiting intent, with reference to the attached figures, in which:
-- figure 1 illustrates in diagram form the device according to the invention as it is used;
-- figures 2 and 3 illustrate, in section, two embodiments of the device according to the present invention;
-- figure 4 illustrates, in section and at enlarged scale, a nano-capacitor used in the device according to the present invention.

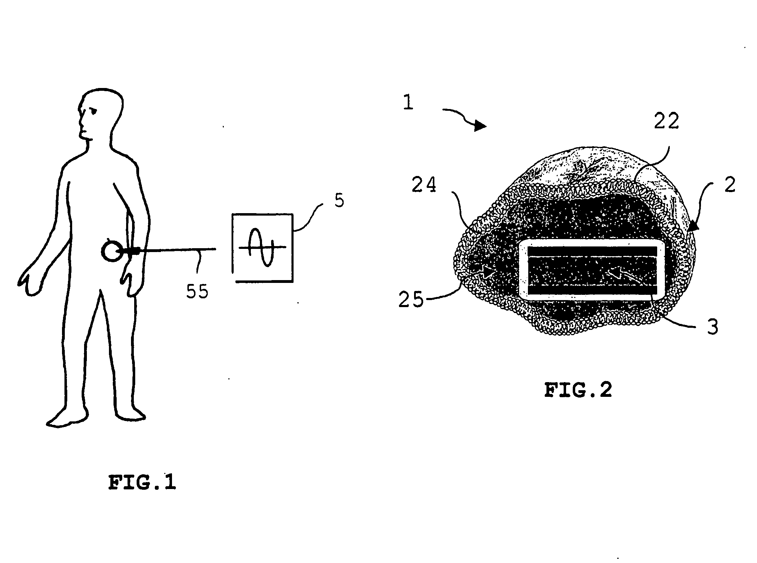

In figure 1, reference 5 indicates overall the source of an alternating electromagnetic field, whereby electromagnetic radiation 55 is sent in the direction of an area of the human body where the drug in question must be released.

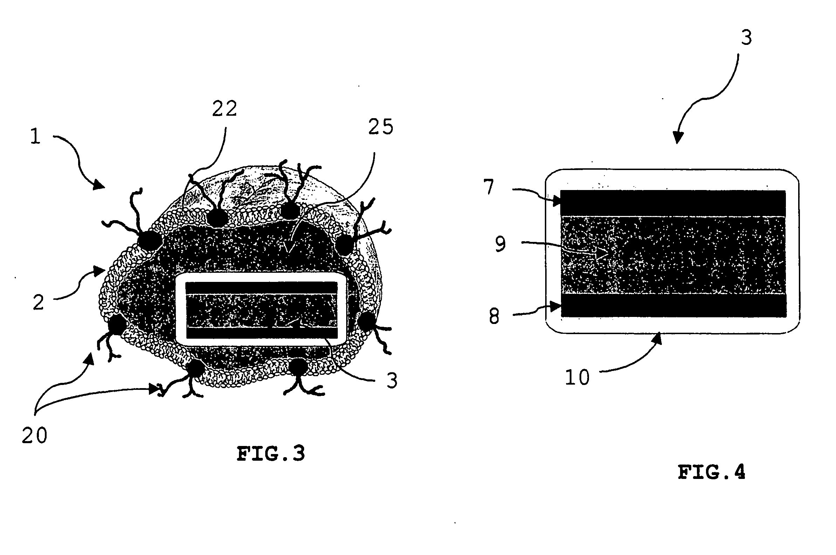

Two embodiments of the device according to the present invention that are preferred at present are represented in figures 2 and 3. The device, indicated overall with reference 1, essentially comprises a membrane 2 having an outer surface 22 and an inner surface 24 and delimiting an enclosed space 25 within which are present at least one micro- or nano-capacitor 3 and at least one drug.

Application from outside through appropriate means 5 of vibrations or heat causes an electric field to be generated by the nano-capacitor 3 with consequent electroporation of the membrane 2 and release of the drug.

In an embodiment of the present invention that is particularly preferred, the membrane 2 is a membrane with single or double phospholipid layer or a liposomal membrane.

On the membrane 2 of the device 1 it is also possible to conjugate molecules 20 (for example proteins, monoclonal antibodies, etc.) having strong affinity for certain proteins (receptors, extra-cellular proteins, etc.) present on the outer surface of the cells to which the treatment is targeted (for example tumour cells), so as to facilitate transport of the device 1 towards the region of interest.

The nano-capacitor 3, in a possible embodiment illustrated in figure 4, essentially comprises an electric nano-condenser including two layers 7 and 8 acting as electrodes between which is interposed a ferroelectric body 9 of the pyroelectric type. In this case the means 5 to generate an electric potential at the electrodes 7 and 8 of said nano-condenser 3 consist of a source of alternating electromagnetic field appropriate to cause, from outside the human or animal body, an increased temperature of said pyroelectric layer, with consequent generation of an electric potential at the electrodes 7 and 8 of the micro-condenser 3.

In a different embodiment, the nano-condenser 3 comprises a ferroelectric body 9 of the piezoelectric type; in this case the means 5 to generate an electric potential at the electrodes 7 and 8 of said nano-condenser 3 comprise a source of vibrations appropriate to cause, from outside the human or animal body, vibrations of the piezoelectric body 9 with consequent generation of an electric potential at the electrodes 7 and 8 of the micro-condenser 3.

In a further embodiment, the nano-capacitor 3 comprises a single-crystal, single-domain ferroelectric body 9 such that generation of an electric potential by the nano-capacitor 3 takes place using the same means 5 utilised in the previous case, but without the requirement for conducting electrodes on the surface of the crystal.

The nano-capacitor 3 may, furthermore, be entirely coated with an outer coating layer 10, for example to reduce rejection phenomena by the human body.

In a further embodiment of the drug delivery device, it is possible to disperse within the space 25 inside the membrane 2 or within the coating layer 10 of the nano-capacitor 3 magnetic particles, superparamagnetic particles or single domain particles (of iron or iron oxide: magnetite or ferrite) for the purpose of improving traceability over time or influencing movement of the device within the human or animal body or, again, associating treatment of the hyperthermal type with the drug treatment.

In the case in which superparamagnetic nanoparticles are used, having a diameter generally between 1 and 20 nm, to achieve traceability of the particles and thus of the device 1 the action of an external magnetic field is required to magnetise the particles along a predetermined direction. In the case in which single-domain nanoparticles are used (with diameter between 15 and 40 nm) the action of an external magnetic field is only necessary if the traceability system requires the magnetic moments of the individual particles to be oriented along a reference axis.

Use of magnetic nanoparticles, in particular of particles with non-zero magnetic histeresis (principally single-domain particles) may also aid in the process of pyrolosis of the membrane, and thus the gradual and local release of the drug. It is indeed known that with magnetic nanoparticles subjected to variable magnetic fields, re-magnetisation of the particles translates into an increase in the temperature of the individual particles. Such a temperature increase of the magnetic particles may alsoin association with localised drug release -- give rise to localised hyperthermal treatment.

### EXAMPLES

### Examples 1. Process to incorporate a nano-capacitor inside a liposomal structure

This process is similar to the incorporation of proteins in liposomes.

The initial lipid solution (for example the commercially-available dioleoil phosphatidyl choline) is cold-dried in liquid nitrogen, followed by a further drying process under vacuum. The initial lipid solution comprises phospholipids or sphingolipids (of natural origin) and nonionic surface-active agents (of synthetic origin). The following phospholipids are usually selected for the preparation of liposomes: phosphatidyl choline, phosphatidyl serine, dipalmitoil phosphatidyl choline, hydrogenated phosphatidyl choline, dioleoil phosphatidyl choline. A buffer or water is subsequently added to bring the solution to the required volume, and the organic solvent of the lipid solution is removed by low-pressure evaporation. Maintaining the system in rotation, a lipid film is formed, and this is further dried using a mechanical pump. Subsequently this lipid film is rehydrated with a specific buffer (e.g. N-Tris(hydroxymethyl)methyl-2-aminoethanesulphonic acid, TES) and at the same time the drug of interest is added, preferably in liquid form (for example in solution) as is the nano-capacitor. The mixture is then subjected to mechanical stirring and lastly, by sonication, liposomes are formed containing the drug of interest and the nano-capacitor.

To reduce the problem of rejection of the liposomes by the human body as far as possible, these may be coated with a film of polyethylene glycol (PEG). In this way, the liposomes may circulate freely in the patient's bloodstream without being destroyed; they can remain in the bloodstream for as much as two or three weeks without opening and gradually enter into the cancerous cells through the tumour capillaries: it is known that newly-formed capillaries, destined to supply the tumour area with blood, are more permeable than those of healthy tissues, and thus they favour an accumulation of the liposomes in the neoplastic tissue, where they release the drug, which selectively carries out its toxic action on the cancer cells.

### Example 2. Manufacture of a nano-condenser

As pyroelectric or piezoelectric materials for the layer 9 of the nano-condenser 3, ceramic materials or ferroelectric polymers may for example be used, such as KNN or biocompatible PVDF. Manufacture is preferably achieved through a multi-layer deposition technique followed by photo-lithography and etching. The first layer deposited on a substratum is a sacrificial layer, which is followed by an electrode, then by the ferroelectric layer (with marked pyroelectric or piezoelectric properties) and a second electrode. The electrodes are necessary for polarisation of the material, but they might also be avoided in the final nano-condenser, if a single-crystal, single-domain piezoelectric material is used, that can be excited by means of an external source of vibrations. If excitation is of the electromagnetic type it is however necessary to have at least one metal layer that acts as micro-antenna. The presence of a metal electrode may also be of help for its "functionalisation" that is its capability to link to the active principal comprising the drug.

The control parameters (means of excitation, frequency, number of impulses, length of impulses) may vary for the purpose of determining optimal conditions for effective therapy.

## Claims

1. An intravascular device (1) that can be injected into the bloodstream of a human or animal body, to deliver at least one drug into said human or animal body, comprising:
-- at least one nano-capacitor (3) comprising at least one ferroelectric body (9),
- means (5) to generate, from outside the human or animal body, an electric potential through said nano-capacitor (3); and
-- at least one drug, which is released from said device following the generation of said electric potential,
- wherein the ferroelectric body (9) is of the piezoelectric type, and
- wherein said means (5) to generate the electric potential include a source of vibrations appropriate to cause, from outside the human or animal body, a vibration of the piezoelectric material of said body (9), with consequent generation of an electric potential through said nano-capacitor,
**characterized in that** said device further comprises a membrane (2) having an outer surface (22) and an inner surface (24), where said inner surface (24) defines a closed space (25) inside said membrane (2), and
**in that** said at least one drug and said at least one nano-capacitor (3) are contained in the closed space (25) inside said membrane (2),
so that said electric potential generated through said nano-capacitor (3) by the vibrations applied by said means (5) determines the electroporation of said membrane (2), that is the opening/rupture of parts of the membrane (2) with consequent release of said at least one drug from said membrane (2).

2. Device according to claim 1, **characterised in that** said membrane (2) is constituted of phospholipids.

3. Device according to claim 1 or claim 2, **characterised in that** said membrane (2) presents a single or a double layer structure.

4. Device according to any of the claims from 1 to 3, **characterised in that** said membrane (2) is of the liposomal type.

5. Device according to any of the claims from 1 to 4, **characterised in that** said membrane (2) presents on its outer surface (24) at least one molecule (20) having affinity for a protein present on the outer surface of a cell of interest in the human or animal body.

6. Device according to any of the above claims, **characterised in that** said ferroelectric body (9) also possesses ferromagnetic properties.

7. Device according to claim 6, **characterised in that** said ferroelectric body (9) is comprised of ferroelectric-ferromagnetic composite materials.

8. Device according to any of the above claims, **characterised in that** said nano-capacitor (3) includes two layers (7,8) acting as electrodes, between which is interposed said ferroelectric body (9).

9. Device according to any of the above claims, **characterised in that** the nano-capacitor (3) is provided with an insulating coating (10).

10. Device according to any of the above claims, **characterised in that** the nano-capacitor (3) presents a maximum dimension not above 10 µm, preferably between 100 nm and 1 µm.

11. Device according to any of the above claims, **characterised in that** said device also includes magnetic particles, superparamagnetic particles or single-domain particles dispersed in the closed space (25) inside the membrane (2).

12. Device according to any of the above claims, **characterised in that** said device also includes magnetic particles, superparamagnetic particles or single-domain particles dispersed inside said coating layer (10) of said nano-capacitor (3).

13. Pharmaceutical preparation comprising a plurality of intravascular devices (1) according to any of the claims from 1 to 12 in a pharmaceutically acceptable vehicle.

## Patentansprüche

1. Intravaskuläre Vorrichtung (1), die in den Blutkreislauf eines menschlichen oder tierischen Körpers injiziert werden kann, um wenigstens ein Arzneimittel in den menschlichen oder tierischen Körper abzugeben, aufweisend:
wenigstens einen Nanokondensator (3), der wenigsten einen ferroelektrischen Körper (9) aufweist,
Mittel (5), um von außerhalb des menschlichen oder tierischen Körpers ein elektrisches Potential durch den Nanokondensator (3) zu erzeugen; und
wenigstens ein Arzneimittel, welches freigegeben wird von der Vorrichtung auf die Erzeugung des elektrischen Potentials hin,
wobei der ferroelektrische Körper (9) vom piezoelektrischen Typ ist, und
wobei die Mittel (5), die das elektrische Potential erzeugen, eine Quelle von Schwingungen beinhalten, welche geeignet ist, von außerhalb des menschlichen oder tierischen Körpers, eine Schwingung des piezoelektrischen Materials vom Körper (9) auszulösen, mit nachfolgender Erzeugung eines elektrischen Potentials durch den Nanokondensator,
**dadurch gekennzeichnet, dass** die Vorrichtung weiterhin enthält eine Membran (2), die eine äußere Oberfläche (22) und eine innere Oberfläche (24) hat, wo die innere Oberfläche (24) einen abgeschlossenen Raum (25) innerhalb der Membran (2) definiert, und
in welcher das wenigstens eine Arzneimittel und der wenigstens eine Nanokondensator (3) in dem abgeschlossenen Raum (25) innerhalb der Membran (2) enthalten sind,
so dass das elektrische Potential, welches durch den Nanokondensator (3) erzeugt wird durch die Schwingungen, die durch die Mittel (5) aufgebracht werden, die Elektroporation der Membran (2) bestimmt, d.h. das Öffnen/Einreißen von Teilen dieser Membran (2) mit anschließender Freigabe des wenigstens einen Arzneimittels durch die Membran (2).

2. Vorrichtung nach Anspruch 1, **dadurch gekennzeichnet, dass** die Membran (2) aus Phospholipiden gebildet ist.

3. Vorrichtung nach Anspruch 1 oder 2, **dadurch gekennzeichnet, dass** die Membran (2) eine einfache oder zweifache Schichtenstruktur zeigt.

4. Vorrichtung nach irgendeinem der Ansprüche 1 bis 3, **dadurch gekennzeichnet, dass** die Membran (2) aus dem liposomalen Typ besteht.

5. Vorrichtung nach irgendeinem der Ansprüche 1 bis 4, **dadurch gekennzeichnet, dass** die Membran (2) auf ihrer äußeren Oberfläche (24) wenigstens ein Molekül (20) aufweist, welches Affinität zu einem Protein aufweist, das auf der äußeren Oberfläche einer interessierenden Zelle im menschlichen oder tierischen Körper vorliegt.

6. Vorrichtung nach irgendeinem der vorhergehenden Ansprüche, **dadurch gekennzeichnet, dass** der ferroelektrische Körper (9) auch ferromagnetische Eigenschaften aufweist.

7. Vorrichtung nach Anspruch 6, **dadurch gekennzeichnet, dass** der ferroelektrische Körper (9) aus ferroelektrisch-ferromagnetischem Verbundmaterial zusammengesetzt ist.

8. Vorrichtung nach irgendeinem der vorhergehenden Ansprüche, **dadurch gekennzeichnet, dass** der Nanokondensator (3) zwei Schichten (7, 8) beinhaltet, die als Elektroden wirken, zwischen denen der ferroelektrische Körper (9) eingeschoben ist.

9. Vorrichtung nach irgendeinem der vorhergehenden Ansprüche, **dadurch gekennzeichnet, dass** der Nanokondensator (3) mit einer isolierenden Deckschicht (10) versehen ist.

10. Vorrichtung nach irgendeinem der vorhergehenden Ansprüche, **dadurch gekennzeichnet, dass** der Nanokondensator (3) eine maximale Abmessung hat, die nicht über 10 µm, bevorzugt zwischen 100 nm und 1 µm liegt.

11. Vorrichtung nach irgendeinem der vorhergehenden Ansprüche, **dadurch gekennzeichnet, dass** die Vorrichtung auch magnetische Partikel, superparamagnetische Partikel oder Einzeldomänenpartikel aufweist, die in dem geschlossenen Raum (25) innerhalb der Membran (2) eingestreut sind.

12. Vorrichtung nach irgendeinem der vorhergehenden Ansprüche, **dadurch gekennzeichnet, dass** die Vorrichtung auch magnetische Partikel, superparamagnetische Partikel oder Einzeldomänenpartikel beinhaltet, die innerhalb der Deckschicht (10) des Nanokondensator (3) eingestreut sind.

13. Pharmazeutische Verabreichung, welches eine Mehrzahl von intravaskulären Vorrichtungen (1) nach einem der Ansprüche 1 bis 12 in einem pharmazeutisch verträglichen Trägerstoff enthält.

## Revendications

1. Dispositif intravasculaire (1) qui peut être injecté dans le flux sanguin d'un corps humain ou animal, pour délivrer au moins un médicament dans ledit corps humain ou animal, comprenant :
- au moins un nano-condensateur (3) comprenant au moins un corps ferroélectrique (9),
- des moyens (5) pour produire, depuis l'extérieur du corps humain ou animal, un potentiel électrique au travers dudit nano-condensateur (3) ; et
- au moins un médicament, qui est libéré depuis ledit dispositif suite à la production dudit potentiel électrique,
où le corps ferroélectrique (9) est du type piézoélectrique, et
où lesdits moyens (5) pour produire le potentiel électrique comprennent une source de vibrations propre à provoquer, depuis l'extérieur du corps humain ou animal, une vibration du matériau piézoélectrique dudit corps (9), avec par voie de conséquence production d'un potentiel électrique au travers dudit nano-condensateur,
**caractérisé en ce que** ledit dispositif comprend en outre une membrane (2) avec une surface extérieure (22) et une surface intérieure (24), où ladite surface intérieure (24) définit un volume clos (25) à l'intérieur de ladite membrane (2), et
**en ce que** ledit au moins un médicament et ledit au moins un nano-condensateur (3)sont contenus dans le volume fermé (25) à l'intérieur de ladite membrane (2),
de sorte que ledit potentiel électrique produit au travers dudit nano-condensateur (3) par les vibrations appliquées par lesdits moyens (5) détermine l'électroporation de ladite membrane (2), à savoir l'ouverture/rupture de parties de la membrane (2) avec par voie de conséquence libération dudit au moins un médicament depuis ladite membrane (2).

2. Dispositif selon la revendication 1, **caractérisé en ce que** ladite membrane (2) est constituée de phospholipides.

3. Dispositif selon la revendication 1 ou la revendication 2, **caractérisé en ce que** ladite membrane (2) présente une structure simple ou double couche.

4. Dispositif selon l'une quelconque des revendications 1 à 3, **caractérisé en ce que** ladite membrane (2) est du type liposomal.

5. Dispositif selon l'une quelconque des revendications 1 à 4, **caractérisé en ce que** ladite membrane (2) présente sur sa surface externe (24) au moins une molécule (20) ayant une affinité pour une protéine présente sur la surface externe d'une cellule d'intérêt dans le corps humain ou animal.

6. Dispositif selon l'une quelconque des revendications ci-dessus, **caractérisé en ce que** ledit corps ferroélectrique (9) possède également des propriétés ferromagnétiques.

7. Dispositif selon la revendication 6, **caractérisé en ce que** ledit corps ferroélectrique (9) est formé de matériaux composites ferroélectriques-ferromagnétiques.

8. Dispositif selon l'une des revendications ci-dessus, **caractérisé en ce que** ledit nano-condensateur (3) comprend deux couches (7, 8) jouant le rôle d'électrodes, entre lesquelles est interposé ledit corps ferroélectrique (9).

9. Dispositif selon l'une des revendications ci-dessus, **caractérisé en ce que** le nano-condensateur (3) est pourvu d'un revêtement isolant (10).

10. Dispositif selon l'une des revendications ci-dessus, **caractérisé en ce que** le nano-condensateur (3) présente une dimension maximale non supérieure à 10 µm, de préférence entre 100 nm et 1 µm.

11. Dispositif selon l'une des revendications ci-dessus, **caractérisé en ce que** ledit dispositif comprend également des particules magnétiques, des particules supraparamagnétiques ou des particules à domaine unique dispersées dans le volume clos (25) à l'intérieur de la membrane (2).

12. Dispositif selon l'une des revendications ci-dessus, **caractérisé en ce que** ledit dispositif comprend également des particules magnétiques, des particules supraparamagnétiques ou des particules à domaine unique dispersées à l'intérieur de ladite couche de revêtement (10) dudit nano-condensateur (3).

13. Préparation pharmaceutique comprenant une pluralité de dispositifs intravasculaires (1) selon l'une quelconque des revendications 1 à 12 dans un véhicule pharmaceutiquement acceptable.
